# FASCICULE DE BREVET EUROPEEN

(11) **EP 2 204 154 B1**
(45) Date de publication et mention de la délivrance du brevet: **12.10.2016**
(21) Numéro de dépôt: 09181014.3
(22) Date de dépôt: 30.12.2009
(51) Int. Cl.: A61K 8/02, A61K 8/60, A61Q 19/08

(54) **Association de monosaccharides et d'adénosine et son utilisation en cosmétique**
Kombination von Monosacchariden und Adenosin und deren kosmetischenVerwendung
Association of monosaccharides and adenosine and its cosmetic use

(30) Priorité: 30.12.2008 FR 0859151; 15.01.2009 US 144756 P
(43) Date de publication de la demande: 07.07.2010
(73) Titulaire: L'Oréal, 75008 Paris (FR)
(72) Inventeur: Laboureau, Julien, 92130, Issy Les Moulineaux (FR); Simonnet, Jean-Thierry, 94230, Cachan (FR); Portes, Pascal, 94130, Nogent sur Marne (FR)
(74) Mandataire: Tezier Herman, Béatrice

(56) Documents cités:
- EP-A- 1 424 064
- EP-A- 1 428 522
- EP-A- 1 847 547
- EP-A1- 1 559 417
- FR-A- 2 900 572

## Description

La présente invention concerne une composition notamment cosmétique et/ou dermatologique, comprenant dans un milieu physiologiquement acceptable, l'association d'au moins un monosaccharide choisi parmi le mannose, le rhamnose et leur mélange, et d'au moins un composé additionnel choisi parmi l'adénosine, un de ses analogues et leur mélange.

La peau humaine est constituée de deux couches principales que sont le derme et l'épiderme qui recouvre le derme de manière superficielle. L'épiderme humain naturel est composé principalement de trois types de cellules que sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé « fonction barrière ».

L'épiderme est un épithélium pavimenteux stratifié kératinisé constitué à 90% de kératinocytes. La différenciation progressive des cellules de la membrane basale, qui sépare le derme de l'épiderme, vers la surface de l'épiderme comprend notamment la différenciation des kératinocytes qui migrent vers la surface de la peau où ils desquament.

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. La cohésion entre l'épiderme et le derme est assurée par la jonction dermo-épidermique. C'est une région complexe d'une épaisseur de 100 nm environ qui comprend le pole basal des kératinocytes basaux, la membrane épidermique et la zone sous-basale du derme superficiel.

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Par ailleurs, les collagènes ne sont pas tous synthétisés par les mêmes types cellulaires, les collagènes de type I et III sont essentiellement produits par le fibroblaste dermique alors que le collagène de type VII est produit par deux catégories de cellules, les kératinocytes et les fibroblastes. La régulation de leur expression diffère d'un collagène à un autre, par exemple, les collagènes I et VII ne sont pas régulés de la même manière par certaines cytokines, en effet, le TNF alpha et la leukoréguline stimulent le collagene VII et régulent négativement le collagène I. Enfin, toutes les molécules de collagène sont des variantes d'un précurseur commun qui est la chaîne alpha du procollagène.

Avec le vieillissement, le collagène s'amincit et des rides apparaissent à la surface de la peau. Le vieillissement cutané est un mécanisme génétiquement programmé.

Par ailleurs, certains facteurs d'environnement comme le tabagisme et surtout l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du collagène de type I, apparaît comme un moyen efficace pour pallier les signes du vieillissement cutané. Au cours du vieillissement chronologique et/ou actinique, l'épiderme subit également de nombreuses modifications et dégradations qui se traduisent, avec l'âge, par une altération du microrelief, une altération de la fonction barrière de la peau, une apparition de rides et ridules, une altération des propriétés mécaniques de la peau, notamment un manque d'élasticité de la peau, et une perte d'éclat du teint.

Les rides d'expression sont la résultante de mécanismes différents de ceux générant les rides dues au vieillissement. Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, les seuls moyens couramment utilisés pour agir sur les rides d'expression sont, d'une part, la toxine botulique qui est notamment injectée dans les rides de la glabelle (voir J.D. Carruters et al., J. Dermatol. Surg. Oncol., 1992, 18, pp. 17-21) et, d'autre part, des implants dégradables à base de collagène, d'acide hyaluronique ou d'acide polylactique.

On comprend donc l'importance de pouvoir disposer de compositions dont les effets visent à régénérer le tissu de la peau via une augmentation de la prolifération des kératinocytes, une stimulation de la prolifération et/ou du métabolisme des fibroblastes, avec notamment une stimulation de la synthèse des procollagènes et collagènes ; et aussi à lutter contre les contractions cutanées responsables de la formation des rides d'expression.

Il est connu de la littérature l'utilisation d'agents tels que le rétinol qui favorisent la prolifération des kératinocytes et permettent de stimuler le renouvellement de l'épiderme, maintenir et/ou augmenter l'épaisseur de l'épiderme, on parle alors d'effet biologique direct. Cependant, le rétinol présente un certain nombre de désavantages lors de son utilisation dans une composition cosmétique. En effet, il a une faible stabilité vis-à-vis de l'oxydation et génère des effets secondaires indésirables pour les consommateurs, tels que notamment l'irritation de la peau. Il existe donc un besoin de trouver d'autres composés présentant un effet biologique direct qui soient facilement accessibles et qui présentent une efficacité acceptable pour une utilisation optimale en cosmétique.

La demanderesse a découvert, de manière surprenante et inattendue, que l'association d'au moins un monosaccharide choisi parmi le mannose, le rhamnose et leur mélange, et d'au moins un composé additionnel choisi parmi l'adénosine, un de ses analogues et leurs mélanges, entraîne une complémentarité d'action à la fois sur le microrelief de la peau, en permettant notamment de lutter contre la formation des rides d'expression, mais aussi sur la stimulation de la régénération du derme et/ou de l'épiderme en stimulant le métabolisme et le processus de renouvellement épidermique, ce qui entraîne une diminution de l'apparition des signes du chronovieillissement et du photovieillissement. Ainsi, l'association selon l'invention provoque une relaxation, une décontration et une diminution de la différenciation des cellules contractiles dermiques impliquées dans la genèse des rides d'expression, notamment des fibroblastes situés le long des lignes de tension créées sous l'effet des contractions des muscles peauciers. L'association selon l'invention permet aussi de stimuler de façon synergique la production de procollagène de type I par les fibroblastes dermiques.

La demanderesse a en effet démontré l'activation de la prolifération des kératinocytes et des fibroblastes et la stimulation de la synthèse du procollagène I par le mannose ou le rhamnose. L'utilisation de compositions les contenant permet ainsi de contrer les signes du vieillissement cutané, et en particulier l'atrophie épidermique et/ou dermique liée au vieillissement.

L'utilisation de ces monosaccharides était restée jusqu'alors inconnue pour les effets biologiques directs exposés plus haut. La demande WO 2007/128939 mentionne toutefois une activité anti-âge obtenue par un effet biomécanique d'un agent tenseur en association avec des composés saccharidiques, qui permettent d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs. De même, la demande FR2900572 décrit l'utilisation conjointe d'une composition cosmétique comprenant des composés saccharidiques et d'un dispositif destiné à appliquer des contraintes mécaniques sur la peau, afin d'en améliorer l'homéostasie. Comme décrit précédemment, il s'agit de la combinaison d'une action biologique et d'une action mécanique, cette dernière étant destinée à tendre et contraindre la peau.

La demande WO 2005/063194 décrit une base galénique à très haute tolérance comprenant notamment du mannose ou du rhamnose. Il est spécifié qu'une telle base galénique ne peut fonctionner qu'en association avec un actif dont elle est seulement le véhicule. Les bases galéniques dermiques et/ou cosmétiques divulguées reposent essentiellement sur la présence des deux polyols que sont le mannitol et le xylitol.

D'autre part, l'influence de l'adénosine et d'analogues de l'adénosine sur l'amélioration de l'aspect de la peau est décrite dans les demandes EP 1424064 et EP 1428522. En particulier, il y est spécifié que l'adénosine permet de relaxer ou décontracter les cellules contractiles dermiques qui sont supposées être impliquées dans la genèse des rides d'expression.

La présente invention concerne donc une composition notamment cosmétique et/ou dermatologique, comprenant dans un milieu physiologiquement acceptable, l'association d'au moins un monosaccharide choisi parmi le mannose, le rhamnose et leur mélange, et d'au moins un composé additionnel choisi parmi l'adénosine, un de ses analogues et leur mélange.

Les monosaccharides selon l'invention sont soit la forme D ou la forme L du mannose et/ou du rhamnose, chaque forme pouvant elle-même être l'anomère alpha et/ou béta. Les formes préférées selon l'invention sont le D-mannose et le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners® et Symrise. Dans la présente invention, le monosaccharide est plus spécifiquement sous forme de monomère.

L'adénosine, au sens de la présente invention, est le nucléoside issu de la condensation de l'adénine sur le ribose (sous forme de ribofuranose) via une liaison β-N₉glucoside. L'adénosine joue un rôle important dans les processus biochimiques, tels que le transfert d'énergie lorsqu'elle est, par exemple, sous forme d'adénosine triphosphate (ATP) et/ou d'adénosine diphosphate (ADP) ; ainsi que dans la transduction de signaux lorsqu'elle est, par exemple, sous forme d'adénosine monophosphate cyclique ou AMPc. L'adénosine présente la formule développée suivante :

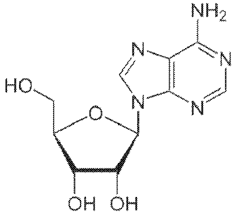

Parmi les analogues d'adénosine utilisables selon l'invention, on citera notamment les agonistes des récepteurs à l'adénosine et les composés augmentant les niveaux intra ou extracellulaires d'adénosine.

Des exemples d'analogues d'adénosine comprennent : la 2'-deoxyadénosine ; la 2', 3'-isopropylidene adénosine; la toyocamycine ; la 1-méthyladénosine ; la N-6-methyladénosine ; l'adénosine N-oxyde; le 6-méthylmercaptopurine riboside et le 6-4 chloropurine riboside.

D'autres analogues d'adénosine comprennent les agonistes des récepteurs à l'adénosine dont la phénylisopropyl-adénosine ("PIA"), la 1-méthylisoguanosine, la N6-Cyclohexyladénosine (CHA), la N6-cyclopentyladénosine (CPA), la 2-chloro-N6- cyclopentyladénosine, la 2-chloroadénosine, la N6-phényladénosine, la 2-phénylaminoadénosine, la MECA, la N6-phénéthyladénosine, la 2-p-(2-carboxy-éthyl) phénéthyl-amino-5'-N-éthylcarboxamido-adénosine (CGS-21680), la N éthylcarboxamido-adénosine (NECA), la 5' (N-cyclopropyl)-carboxamidoadénosine, la DPMA (PD 129,944) et le metrifudil.

Parmi les analogues d'adénosine augmentant la concentration intracellulaire d'adénosine dans la composition selon l'invention, sont préférés les composés compris dans le groupe formé par l'érythro-9-(2-hydroxy-3-nonyl) adénine ("EHNA") et l'iodotubercidine.

Les analogues de l'adénosine peuvent être également des composés de formule (I) suivante : 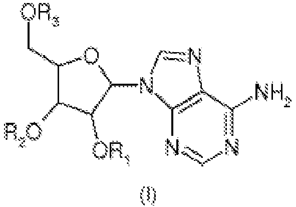 dans laquelle :
R1 et R2 identiques désignent un radical hydrocarboné linéaire saturé en C1-C6 ou insaturé en C2-C6, ou ramifié en C3-C6 saturé ou insaturé, ou bien encore forment ensemble, avec les atomes d'oxygène auxquels ils sont rattachés, un radical isopropylidène ;
R3 désigne :
   (i) un radical hydrocarboné linéaire saturé en C1-C10 ou insaturé en C2-C10, ou ramifié en C3-C10 saturé ou insaturé, éventuellement substitué par au moins un groupement choisi parmi -OR', -NR'R", -COOR', -CONR'R", -CF3, -F, -OCF3, -CN, -NO2, ou
   (ii) un groupe -COR4 avec R4 désignant un radical hydrocarboné linéaire saturé en C1-C9 ou insaturé en C2-C9, ou ramifié en C3-C9 saturé ou insaturé, éventuellement substitué par au moins un groupement choisi parmi -OR', -NR'R", -COOR', -CONR'R", -CF3, -F, -OCF3, -CN, -NO2 ou
   (iii) un groupe ester issu de la biotine;
      R' et R" désignant un atome d'hydrogène, un radical hydrocarboné linéaire saturé en C1-C6 ou insaturé en C2-C6, ou ramifié en C3-06 saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en C1-C6 ou insaturé en C2-C6, ou ramifié en C3-C6 saturé ou insaturé ; et
      leurs sels, isomères optiques et solvates.

Ces composés sont notamment décrits dans la demande de brevet FR 2899584 déposée par l'Oréal.

Un radical hydrocarboné est avantageusement un radical alkyle saturé ou insaturé, linéaire ou ramifié. Parmi les groupes alkyles convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Plus préférentiellement, on utilise des composés de formule (I) pour lesquels : R1 et R2 forment ensemble, avec les atomes d'oxygène auxquels ils sont rattachés, un radical isopropylidène, R3 désigne : un groupe -COR4 avec R4 désignant un radical hydrocarboné linéaire en C1-C9 ; ou un groupe ester issu de la biotine.

Le 2',3'-isopropylidène-5'-acétyl-adénosine est un composé connu, notamment décrit dans la demande WO-A-2004/037159 (composé 265 page 203) dans une composition pharmaceutique pour le traitement de l'obésité. Ce document ne décrit pas d'appliquer la composition par voie topique sur la peau, notamment pour traiter les rides.

Certains des composés de formule (I) sont connus de l'art antérieur et décrits dans les documents suivants :
- WO-A-2004/037159 ;
- Poppe, L et al ; "Synthesis and characterization of (5'-deoxyadenosin-5'-yl)cobalamin (='adenosylcobalamin') analogs mimicking the transition-state geometry of coenzyme-B12-dependent rearrangements" ; Helvetica Chimica Acta (1993), 76(6), 2367-83 ;
- Jones, A. S. et al ; "Synthetic analogs of polynucleotides. VII. Syntheses of 5'-O-acryloylnucleosides and copolymers of these with other acryloyl compounds" ; Journal of the Chemical Society [Section] C: Organic (1971), (19), 3183-7 ;

- Mornet, D. et al ; "The reaction of myosin with a bromoalkyl analog of adenosine 40 triphosphate" ; FEBS Letters (1977), 84(2), 362-6 ; 4 25 30 - Huber Gerhard ; "esters of adenosine with organic and inorganic" acids; Chem. Ber. 89, 2853-62 (1956) - ref CA52:2027g.
- Takemoto, K. et al; "Nucleic acid analogs: their specific interaction and applicability"; 5 Polymeric Materials Science and Engineering (1988), 58, 250-3;
- Purkayastha, Bhupesh C.; Bhattacharyya, S. N ; "Use of Ca oxalate monohydrate in the investigation of rare earth and thorium activities" ; J. Indian. Chem. Soc. 34, 427-33 (1957) - ref CA52:2627h;
- Peterli, Stefan et al ; "Nitrostyrene derivatives of adenosine 5'-glutarates as selective inhibitors of the epidermal growth factor receptor protein tyrosine kinase" ; Helvetica Chimica Acta (1992). 75(3), 696-706.

A titre de composés de formule (I), on peut notamment citer les composés suivants : 2',3'-isopropylidène-5'-butanoyle-adénosine, 2',3'-isopropylidène-5'-octanoyle-adénosine, 2',3'-isopropylidène-5'-biotinoyle-adénosine, 2',3'-isopropylidène-5'-éthyl-adénosine, 2',3'-isopropylidène-5'-octyle-adénosine, 2',3'-diméthyl-5'-éthyl-adénosine, 2',3'-diméthyl-5'-butanoyle-adénosine, ou 2',3'-isopropylidène-5'-acétyl-adénosine (n° CAS 15888-38-7).

L'adénosine est préférée dans la présente invention. Elle est notamment disponible dans le commerce sous forme de poudre auprès de la société PHARMA WALDHOF.

La présente invention porte aussi sur l'utilisation d'une composition selon l'invention telle que définie précédemment, administrée par voie orale, topique ou par injection cutanée, notamment pour le soin de la peau et/ou du cuir chevelu.

Une composition conforme à l'invention telle que définie précédemment peut notamment être une composition cosmétique pour soin capillaire pour, en particulier, la stimulation de la pousse du cheveu, la lutte contre la chute des cheveux, le ralentissement de sa chute ou le renforcement de l'éclat du cheveu.

La présente invention a également pour objet une méthode de traitement, en particulier cosmétique ou thérapeutique, pour diminuer ou prévenir les signes du vieillissement de la peau ou de ses phanères (cheveux, cils, ongles, ...), par administration à un sujet, de préférence un être humain, d'une quantité efficace d'au moins un monosaccharide tel que défini précédemment en association avec une quantité efficace d'au moins un composé additionnel tel que défini précédemment. L'invention a en particulier pour objet un procédé de traitement cosmétique d'une peau ridée, en particulier la peau du visage et/ou du front, comprenant l'application topique sur ladite peau d'une composition comprenant, dans un milieu physiologiquement acceptable, l'association d'une quantité efficace d'au moins un monosaccharide tel que défini précédemment et d'une quantité efficace d'au moins un composé additionnel tel que défini précédemment.

La composition selon l'invention est plus particulièrement destinée à être appliquée sur les zones du corps, du visage ou du front marquées par des rides, et/ou sur les personnes présentant des rides.

La présente invention concerne également l'utilisation des compositions ou de l'association selon l'invention, pour diminuer et/ou prévenir les signes du vieillissement de la peau ou de ses phanères, en particulier pour diminuer et/ou prévenir les rides de la peau.

Les rides incluent les rides d'expression et celles dues au vieillissement. Les rides d'expression sont de préférence choisies parmi : les rides de la patte d'oie, les sillons nasogéniens, les rides inter-sourcilières et les rides du front.

La composition ou l'association selon l'invention permet aussi de stimuler la régénération des cellules de l'épiderme et du derme, au niveau de la peau ou des phanères, en particulier les kératinocytes et les fibroblastes, notamment par augmentation de leur prolifération. On dispose de la sorte d'une méthode, notamment cosmétique, efficace notamment pour lutter contre les signes du chronovieillissement et/ou du photovieillissement.

Les signes du photovieillissement correspondent aux dégradations internes de la peau consécutives à une exposition aux rayonnements ultra-violets (vieillissement actinique). Les signes du chronovieillissement correspondent aux dégradations internes de la peau dues au vieillissement intrinsèque des individus.

Selon une forme de réalisation préférée, l'utilisation selon la présente invention est destinée à améliorer l'éclat du teint, à diminuer et/ou prévenir les caractéristiques des rides et/ou ridules, à améliorer et/ou diminuer le micro-relief de la peau, et/ou à lisser la peau et/ou à améliorer les propriétés mécaniques de la peau et/ou pour relaxer ou décontracter les cellules contractiles dermiques impliquées dans la genèse des rides d'expression.

Selon un autre aspect de l'invention, l'utilisation de la composition ou de l'association selon l'invention permet d'améliorer la densité de la peau, sa fermeté et/ou la cohésion de ses différents compartiments, en particulier la cohésion du derme avec l'épiderme.

La présente invention concerne également l'utilisation de la composition ou de l'association selon l'invention pour traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie, et/ou les dégradations internes de la peau consécutives à une exposition aux rayonnements ultra-violets.

La présente invention concerne également l'utilisation de la composition ou de l'association selon l'invention pour décontracter la peau et/ou détendre les traits de la peau.

Les compositions ou l'association selon la présente invention ont également pour effet d'augmenter la synthèse des collagènes, de préférence le pro-collagène I.

La quantité des ingrédients actifs, choisis parmi les monosaccharides et l'adénosine et analogues tels que précédemment définis, à mettre en oeuvre selon l'invention dépend de l'effet cosmétique ou thérapeutique recherché, et peut donc varier dans une large mesure. L'homme de l'art peut aisément, sur la base de ses connaissances générales, déterminer les quantités appropriées.

Ainsi, et selon une forme de réalisation préférée, la composition selon l'invention comprend au moins un monosaccharide tel que défini ci-dessus en une quantité comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

Selon une autre forme de réalisation préférée, la composition selon l'invention comprend au moins un monosaccharide tel que défini ci-dessus en une quantité comprise entre 1 % et 10 % en poids, et plus particulièrement entre 1 % et 6 % en poids par rapport au poids total de la composition.

Selon une forme de réalisation préférée, la composition selon l'invention comprend l'adénosine et/ou au moins un de ses analogues en une quantité comprise entre 0,001 % et 10 % en poids par rapport au poids total de la composition, et en particulier entre 0,01% et 3 % en poids, et plus particulièrement entre 0,01 % et 1 % en poids par rapport au poids total de la composition.

Selon une autre forme de réalisation préférée, la composition selon l'invention comprend l'adénosine et/ou au moins un de ses analogues en une quantité comprise entre 0,01 % et 10 % en poids par rapport au poids total de la composition, et en particulier entre 0,01 % et 2 % en poids par rapport au poids total de la composition.

Les monosaccharides, ainsi que l'adénosine, un de ses dérivés ou analogues, présents dans la composition ci-dessus définie sont des agents actifs de la composition.

Par agent actif ou ingrédient actif au sens de la présente invention, on entend plus spécifiquement un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

De manière préférée, la composition selon la présente invention comprend, en outre, au moins un sel de magnésium et au moins un sel de potassium.

L'association de l'adénosine avec un sel de magnésium et un sel de potassium a été décrite dans la demande brevet EP1428522.

Comme sels de magnésium et de potassium, on peut citer les sels organiques et inorganiques de ces métaux.

Des exemples de sels organiques de magnésium et de potassium comprennent les sels formés à partir d'un contre-anion choisi parmi : un anion citrate, oxalate, acétate, gluconate, lactate, tartrate, maléate, benzoate, propionate, salicylate, ascorbate, formate, succinate, folinate, aspartate, phthalate, oléate, palmitate, stéarate, lauryl sulfate, lanolate, myristate, béhénate, caséinate, cyclamate, pantothénate, polyaminopolycarboxylate, thioglycolate, laurate, ricinoléate, pidolate, sorbate ou glycyrrhizinate.

Des exemples de sels inorganiques de magnésium et de potassium comprennent les sels formés à partir d'un contre-anion choisi parmi : un anion nitrate, sulfate, halogénure, carbonate, bicarbonate, hydroxyde, peroxyde, nitrure, sulfure, bisulfate, persulfate, glycérophosphate, hypophosphate ou borate. Le sulfate de magnésium et le glycyrrhizinate dipotassique sont préférés pour une utilisation dans la présente invention.

Les quantités d'adénosine et de sels qui peuvent être introduites dans la composition selon l'invention peuvent varier dans une large mesure en fonction de l'effet recherché. A titre d'exemple, le sel de magnésium peut représenter de 0,001 à 1 %, et de préférence de 0,01 à 0,1% du poids total de la composition. Le sel de potassium peut représenter de 0,001 à 1 %, et de préférence de 0,01 à 0,1 % du poids total de la composition.

La présente invention concerne, notamment, une des compositions telles que définies précédemment, adaptée à une administration topique sur la peau ou ses phanères, une administration orale ou une injection cutanée.

La présente invention concerne aussi une composition selon l'invention, adaptée à une administration topique, se présentant avantageusement sous forme d'une crème, d'un gel, d'une lotion, d'un lait, d'une huile, d'un onguent, d'une cire, d'une mousse, d'une pâte, d'un sérum, d'une pommade ou d'un shampooing.

Lorsque la composition selon la présente invention est administrée par voie orale, elle peut se présenter avantageusement sous la forme d'une gélule, d'un comprimé, ou de pilules. Lorsque la composition selon la présente invention est administrée par injection cutanée, elle peut être en particulier sous la forme d'une solution stérile.

D'une manière générale, le milieu dans lequel sont compris les principes actifs de la composition tel que définie précédemment, est un milieu physiologiquement acceptable, en particulier un milieu cosmétiquement ou pharmaceutiquement acceptable et peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans huile (E/H) ou multiple (triple : E/H/E ou H/E/H), des nanoémulsions, en particulier des nanoémulsions H/E, dont la tailles des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules R1 COOR2 et R1OR2 dans laquelle R1 représente le reste d'un acide gras ou d'unalcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol et le propylène glycol.

Comme gélifiants hydrophiles utilisables dans l'invention, on peut citer à titre d'exemples non limitatifs, les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Les compositions de l'invention peuvent contenir d'autres actifs hydrophiles ou lipophiles. Ces actifs sont notamment choisis parmi les agents anti-oxydants, les agents dermo-relaxants ou dermodécontractants, les agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules. Des listes de ces actifs sont données à la suite à titre illustratif, et ne doivent en aucune façon être considérées comme limitatives.

### Agents anti-âge :

Parmi les actifs connus pour lutter contre les signes du vieillissement, notamment cutané, on peut citer notamment :
la vitamine B3, le coenzyme Q10 (ou ubiquinone), la vitamine B9, la vitamine E, les dérivés de la vitamine E tels que le dérivé phosphaté comme par exemple le TPNA^{®} commercialisé par la société Showa Denko, le resvératrol ou ses dérivés, comme par exemple le resveratrate^{®} commercialisé par la société Estée Lauder, le rétinol ou ses dérivés, et leur mélange.

### Agents anti-glycation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus),* par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE^{®} » par la société SOLABIA, le chorhydrate de carcinine (commercialisé par Exsymol sous la dénomination « ALISTIN^{®}»), un extrait d*'Hélianthus annuus* comme l'Antiglyskin^{®} de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777^{®} de Laboratoires Sériobiologiques, un extrait de thé noir comme le Kombuchka^{®} de Sederma et leurs mélanges.

Comme agents anti-glycation préférés, on citera les extraits de myrtille (*Vaccinium myrtillus*) et l'extrait de thé noir.

### Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :
- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®}, les peptides de riz tel que le Nutripeptide^{®} de SILAB, le méthylsilanol mannuronate tel que l'Algisium C^{®}) commercialisé par Exsymol ; les hormones végétales telles que les auxines et les lignanes ; l'acide folique ; et un extrait de Medicago sativa (alfafa) tel que celui commercialisé par SILAB sous la dénomination Vitanol^{®}; un extrait peptidique de noisette tel que celui commercialisé par la société Solabia sous la dénomination Nuteline C^{®}; et l'arginine.
- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol^{®} de Silab, un extrait d'aphanizomenon flos-aquae (cyanophycée) commercialisée sous la dénomination Lanablue^{®} par Atrium Biotechnologies, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon; un extrait de litchi ; la DIPALMITOYL HYDROXYPROLINE commercialisé par Seppic sous le nom SEPILIFT DPHP^{®} : Baccharis genistelloide ou Baccharine commercialisé par SILAB, un extrait de moringa tel que l'Arganyl LS 9781^{®} de Cognis ; l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.
- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin^{®} ; et l'extrait d'algue *Macrocystis pyrifera* commercialisé par la société SECMA sous la dénomination commerciale Kelpadelie^{®}; un extrait peptidique de noisette tel que celui commercialisé par la société Solabia sous la dénomination Nuteline C^{®}.
- soit sur l'inhibition de la dégradation de l'élastine tels que l'extrait peptidique de graines de *Pisum sativum* commercialisé par la société LSN sous la dénomination commerciale Parelastyl^{®} ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C₁-C₆., un extrait de peptides de riz tel que la Colhibin^{®} de Pentapharm, ou un extrait de Phyllanthus emblica tel que l'Emblica^{®} de Rona.
- soit sur la synthèse des glycosaminoglycannes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; l'extrait de *Saccharomyces cerevisiae* disponible notamment auprès de la société SILAB sous la dénomination commerciale Firmalift^{®} ou auprès de la société LSN sous la dénomination commerciale Cytovitin^{®}-, un extrait de *Laminaria ochroleuca* tel que la Laminaïne^{®} de Secma ; l'essence de Mamaku de Lucas Meyer, un extrait de cresson (Odraline^{®} de Silab).
- soit sur la synthèse de la fibronectine, tels que l'extrait de zooplancton Salina commercialisé par la société SEPORGA sous la dénomination commerciale GP4G^{®} ; l'extrait de levure disponible notamment auprès de la société ALBAN MÜLLER sous la dénomination commerciale Drietine^{®} ; et le palmitoyl pentapeptide commercialisé par la société SEDERMA sous la dénomination commerciale Matrixit^{®}.

Parmi les actifs stimulant les macromolécules épidermiques, telles que la fillagrine et les kératines, on peut citer notamment l'extrait de lupin commercialisé par la société SILAB sous la dénomination commerciale Structurine^{®} ; l'extrait de bourgeons de hêtre *Fagus sylvatica* commercialisé par la société GATTEFOSSE sous la dénomination commerciale Gatutine^{®} RC; et l'extrait de zooplancton Salina commercialisé par la société SEPORGA sous la dénomination commerciale GP4G^{®}; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397^{®}.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer : les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®}; les peptides de riz tel que le Nutripeptide^{®} de SILAB, le méthylsilanol mannuronate tel que l'Algisium C^{®} commercialisé par Exsymol ; l'acide folique ; un extrait de Medicago sativa (alfafa) tel que celui commercialisé par SILAB sous la dénomination Vitanol^{®}; un extrait peptidique de noisette tel que celui commercialisé par la société Solabia sous la dénomination Nuteline C^{®}; l'arginine ; un extrait d'aphanizomenon flos-aquae (cyanophycée) commercialisée sous la dénomination Lanablue^{®} par Atrium Biotechnologies, l'extrait de malt commercialisé par la société COLETICA sous la dénomination commerciale Collalif^{®} le lycopène ; un extrait de litchi ; un extrait de moringa tel que l'Arganyl LS 9781^{®} de Cognis ; un extrait de *vaccinium myrtillus* tel que ceux décrits dans la demande FR-A-2814950 ; le retinol et dérivés en particulier le palmitate de rétinol ; l'extrait de *Saccharomyces Cerivisiae* commercialisé par la société LSN sous la dénomination commerciale Cytovitin^{®} ; un extrait peptidique de noisette tel que celui commercialisé par la société Solabia sous la dénomination Nuteline C^{®}; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C₁-C₆. ; un extrait de peptides de riz tel que la Colhibin^{®} de Pentapharm, ou un extrait de Phyllanthus emblica tel que l'Emblica^{®} de Rona ; l'extrait d'algue brune Padina pavonica commercialisé par la société ALBAN MÜLLER sous la dénomination commerciale HSP3^{®} ; l'extrait de *Saccharomyces cerevisiae* disponible notamment auprès de la société SILAB sous la dénomination commerciale Firmalift^{®} ou auprès de la société LSN sous la dénomination commerciale Cytovitin^{®}-, un extrait de *Laminaria ochroieuca* tel que la Laminaïne^{®} de Secma ; l'essence de Mamaku de Lucas Meyer, l'extrait de lupin commercialisé par la société SILAB sous la dénomination commerciale Structurine^{®} , l'extrait de bourgeons de hêtre *Fagus sylvatica* commercialisé par la société GATTEFOSSE sous la dénomination commerciale Gatuline^{®} RC.

### Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; un extrait de protéines hydrolysées de soja tel que le RIDULISSE^{®} de SILAB ; et les hormones végétales telles que les giberrellines et les cytokinines ; un extrait peptidique de noisette tel que celui commercialisé par la société Solabia sous la dénomination Nuteline C^{®}.

De préférence on utilisera un agent favorisant la prolifération et/ou la différenciation des kératinocytes.

Les agents stimulant la prolifération des kératinocytes, utilisables dans la composition selon l'invention, comprennent notamment ; le phloroglucinol, l'extrait de feuille d'hydrangea macrophylla comme l'Amacha liquid E^{®} d'Ichimaru Pharcos, un extrait de levure tel que le Stimoderm^{®} de CLR ; l'extrait de *Larrea divaricata* tel que le Capislow^{®} de Sederma, les mélanges d'extraits de papaye, de feuilles d'olivier et de citron tel que la Xyléine^{®} de Vincience, l'extrait de feuille d'hydrangea macrophylla comme l'Amacha liquid E^{®} d'Ichimaru Pharcos, le rétinol et ses esters dont le palmitate de rétinyle, les extraits de tourteaux de noix commercialisés par Gattefosse et les extraits de *solanum tuberosum* tel que le Dermolectine^{®} commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine^{®}, le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®}; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol, le rétinol et ses esters dont le palmitate de rétinyl.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; un extrait de protéines hydrolysées de soja tel que le RIDULISSE^{®} de SILAB ; un extrait peptidique de noisette tel que celui commercialisé par la société Solabia sous la dénomination Nuteline C^{®}; l'adénosine, le phloroglucinol, un extrait de levure tel que le Stimoderm^{®} de CLR ; un extrait peptidique de lupin tel que celui commercialisé par la société SILAB sous la dénomination commerciale Structurine^{®}, un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; le rétinol et ses esters dont le palmitate de rétinyl.

### Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance^{®} de National Starch et les autres actifs mentionnés dans la demande L'OREAL FR2877220.

### Inhibiteurs de NO-svnthases

L'agent ayant une action d'inhibiteur de NO synthase peut être choisi parmi les OPC (oligomères procyanidoliques); les extraits de végétal de l'espèce *Vitis vinifera* notamment commercialisés par la société Euromed sous la dénomination Leucocyanidines de raisins extra, ou encore par la société Indena sous la dénomination Leucoselect^{®}, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol^{®} BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

### Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital^{®} de Sederma, le mélange de sels de sodium, de manganèse, de zinc et de magnésium d'acide pyrrolidone carboxylique comme le Physiogenyl^{®} de Solabia, un mélange de gluconate de zinc, de cuivre et de magnésium tel que le Sepitonic M3^{®} de Seppic et leurs mélanges ; et un beta-glucan issu de *Saccharomyces cerevisiae,* tel que celui commercialisé par la société Mibelle AG Biochemistry.

L'invention porte également sur un procédé de traitement cosmétique de la peau destiné à diminuer ou prévenir les signes du vieillissement de la peau ou de ses phanères (cheveux, cils, ongles, ...), comprenant au moins une étape consistant à appliquer sur la peau au moins une composition telle que définie précédemment.

Le procédé selon l'invention comprend plus spécifiquement au moins une étape consistant à appliquer sur la peau de personnes présentant une peau présentant au moins l'un des signes de vieillissement cutané rappelés précédemment au moins une composition telle que définie précédemment.

Plus particulièrement, il comprend au moins une étape consistant à appliquer sur la peau de personnes présentant une peau ou une zone de peau vieillie, ridée, ou molle et/ou flasque ou sur des zones du corps présentant une perte d'élasticité et/ou de fermeté et/ou de tonicité au moins une composition telle que définie précédemment.

La composition selon l'invention peut être appliquée sur la partie de la peau ou des phanères à traiter, en particulier sur le visage, le corps, le cou, les mains, les cheveux ou le cuir chevelu, de préférence de façon quotidienne ou pluriquotidienne. L'application pourra être par exemple renouvelée tous les jours pendant une période variable selon les effets souhaités, généralement de 3 à 6 semaines, mais pourra être prolongée ou poursuivie en continu.

Selon une alternative, la composition selon l'invention peut être administrée par voie injectable en association ou non avec des produits de comblement. En effet, l'une des solutions retenues pour lutter contre les rides et/ou la perte de volume des tissus mous est l'utilisation de produits de comblement (ou "filler"). Ce comblement peut être réalisé par l'utilisation de produits non résorbables, tels que des gels de polyacrylamide ou des particules de polyméthylméthacrylate (PMMA). Néanmoins, ces composés peuvent entrainer des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants. A ce jour, l'acide hyaluronique utilisé dans des formes pharmaceutiques ou des dispositifs médicaux se présente sous la forme d'un gel de hyaluronate de sodium. Le monosaccharide selon l'invention ou les compositions les contenant pourront également être appliqués par mésothérapie. La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004. La mésothérapie faite sur le visage est également appelée mésolift, ou également sous le terme anglosaxon de « mesoglow ».

Ainsi, un autre objet de la présente invention peut être un dispositif, en particulier un dispositif médical, comprenant une quantité efficace d'au moins un monosaccharide tel que défini précédemment en association avec une quantité efficace d'adénosine ou un de ses analogues. Ce dispositif peut être adapté à une injection intraépidermique et/ou intradermique et/ou sous-cutanée. L'association d'actifs telle que définie ci-dessus est mise en solution dans un milieu stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, et au moins l'association d'actifs, monosaccharide(s) et adénosine ou analogue, tels que définis ci-dessus. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ladite l'association d'actifs, monosaccharide(s) et adénosine ou analogue, éventuellement en association avec au moins un autre composé actif, comme au moins un produit résorbable ou non, tel que les produits de comblement mentionnés ci-dessus, éventuellement réticulé.

L'injection de l'association selon l'invention peut être réalisée simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

Selon un autre aspect, l'invention concerne également un ensemble cosmétique comprenant : i) un récipient délimitant au moins un compartiment, ledit récipient étant fermé par un élément de fermeture ; et ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier. L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage.

Alternativement, le couplage entre l'élément de fermeture et le récipient se fait autrement que par vissage, notamment via un mécanisme à baïonnette, par encliquetage, serrage, soudage, collage, ou par attraction magnétique. Par " encliquetage " on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube. Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le contenu des brevets ou demandes de brevets cités précédemment sont incorporés par référence dans la présente demande.

Selon un mode particulier, l'invention concerne un ensemble cosmétique comprenant :
- une composition A contenant au moins un composé choisi parmi l'adénosine, un de ses analogues et leurs mélanges,
- une composition B, conditionnée de manière séparée de la composition A, comprenant au moins un monosaccharide choisi parmi le mannose, le rhamnose et leur mélange.

L'invention concerne enfin un procédé de traitement cosmétique ou dermatologique comprenant au moins une étape d'administration, en particulier d'application topique, sur la peau et/ou ses phanères, de la composition A et au moins une étape d'administration, en particulier d'application topique sur la peau et/ou ses phanères, de la composition B.

L'administration de la composition A selon l'invention peut être réalisée simultanément à, ou avant ou après, l'administration de la composition B. Comme spécifié précédemment, l'administration de la composition A et de la composition B peut être réalisée par voie topique, orale ou par injection.

Selon une alternative, on administre en premier lieu la composition A et en second lieu la composition B. Selon une autre alternative, on administre en premier lieu la composition B et en second lieu la composition A.

Les compositions A et B peuvent être conditionnées séparément à l'intérieur de deux compartiments, formés soit par deux récipients distincts, soit à l'intérieur d'un dispositif unitaire. Par " dispositif unitaire " on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment via un piston apte à coulisser à l'intérieur du récipient, ou via les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

### Légendes des Figures

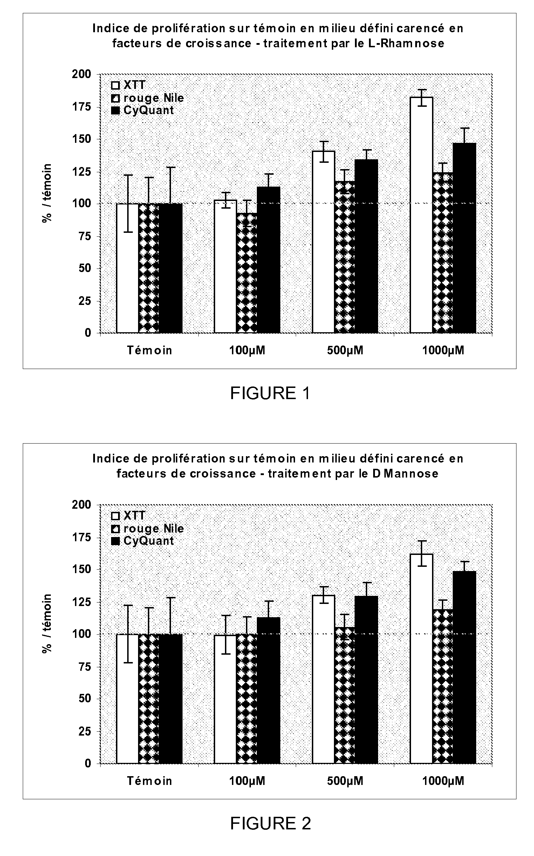

**Figure 1** **:** Diagramme schématisant les résultats obtenus pour la prolifération des kératinocytes, en présence d'un témoin, en présence de différents marqueurs, en milieu carence en facteurs de croissance, et avec ajout de différentes concentrations en L-Rhamnose reportées en abscisse. Les valeurs reportées en ordonnée correspondent aux pourcentages de cellules marquées mesurés par rapport au témoin.
**Figure 2** **:** Diagramme schématisant les résultats obtenus pour la prolifération des kératinocytes, en présence d'un témoin, en présence de différents marqueurs, en milieu carence en facteurs de croissance, et avec ajout de différentes concentrations en D-Mannose reportées en abscisse. Les valeurs reportées en ordonnée correspondent aux pourcentages de cellules marquées mesurés par rapport au témoin.

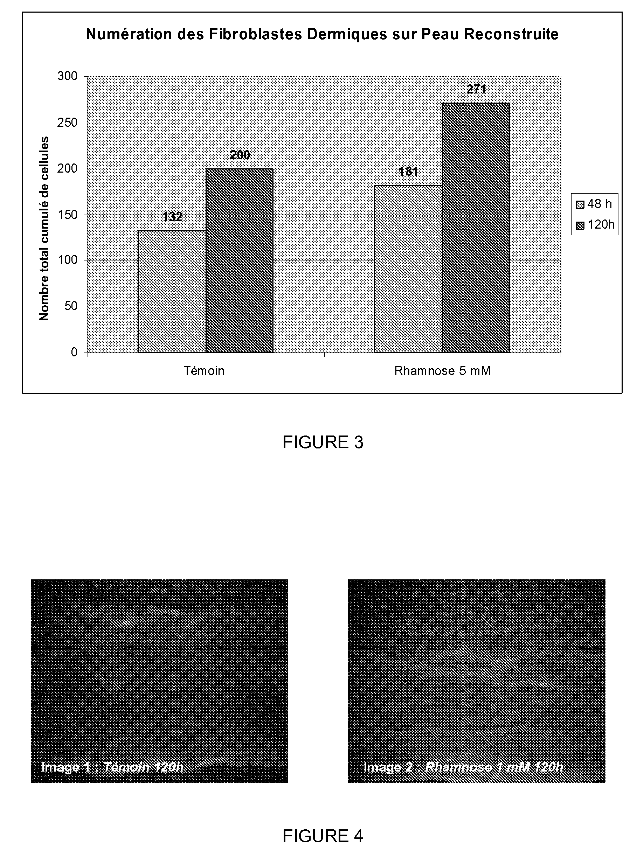

**Figure 3** **:** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.
**Figure 4** **:** photographies de coupe à congélation de peau reconstruite de 7µM d'épaisseur. Le niveau de fluorescence se matérialise par les tâches blanche du cliché en noir et blanc, il est proportionnel à la quantité de procollagène de type I. A gauche figure la peau témoin et à droite la peau traitée par 1 mM de rhamnose.

L'invention est illustrée plus en détail dans les exemples suivants qui sont présentés à titre illustratif et non limitatif du domaine de l'invention.

### Exemples

### Exemple 1 : Prolifération des kératinocytes

### Protocole

Les kératinocytes (lignée HaCat) sont cultivés dans deux conditions : milieu de culture défini complet (condition standard) et milieu de culture carence en facteurs de croissance. Ce milieu carence entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.

La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

### Résultats

Les résultats sont donnés dans les figures 1 et 2.
Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.

Cette activation de la prolifération cellulaire par les deux composés se manifeste par un nombre de cellules plus important par rapport au témoin non traité.
Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 µM, les deux molécules présentent déjà une efficacité.
Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.

Le rhamnose et le mannose présentent donc une efficacité anti âge intéressante en venant booster le renouvellement épidermique et lutter contrer l'atrophie épidermique liée au vieillissement.

### Exemple 2 : Prolifération des fibroblastes

### Protocole

Le rhamnose a été étudié sur un modèle de peau reconstruite complète afin de mesurer son efficacité anti âge au niveau du compartiment dermique. Brièvement, le modèle de peau reconstruite utilisé est celui décrit par Bell et al, (Bell E. et al, The reconstitution of living skin, J Invest Dermatol, 1983, Jul ;81) : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.
La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7µM d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

### Résultats

Les résultats sont donnés à la figure 3.
Il a été constaté que le rhamnose induit la stimulation de la croissance des fibroblastes dermiques de la peau reconstruite dès 48 heures de traitement, stimulation confirmée à 120 h de traitement, avec entre 30 à 35% de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

### Exemple 3 : Synthèse de Procollagène 1

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC *(112-095-068 Jackson Immunoresearch)*)*.* Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image, la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique. Sur l'image 2, figure 4, correspondant par exemple, à une coupe de la peau reconstruite traitée par le Rhamnose à 1 mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien son efficacité anti-âge sur le derme, efficacité complémentaire de celle mesurée vis-à-vis du compartiment épidermique.

### Exemple 4 : Association Rhamnose et adénosine : Mise en évidence de la complémentarité d'action anti âge de l'adénosine et du rhamnose sur la Physiologie cutanée (stimulation de la synthèse du procollagène I par les fibroblastes dermiques).

### 1. Cellules utilisées

Les fibroblastes dermiques humains proviennent de plasties mammaires ou abdominales (pas de différences entre les fibroblastes issus du derme papillaire ou réticulaire). Ils sont utilisés au 9^{ème} passage et cultivés jusqu'à confluence à 37°C et 5% de CO₂ dans un milieu DMEM (Invitrogen 21969035) additionné :
- de Sérum de Veau Foetal (SVF) à 10%,
- de L-glutamine à 2mM (Invitrogen 25030024)
- de Pénicilline à 50 UI/mL
- de Streptomycine à 50 µg/mL (Invitrogen 15070063)
- de pyruvate de sodium (Invitrogen 11360039)
- d'acides aminés non essentiels (Invitrogen 11140035)
Le milieu d'essai des principes actifs est le même que précédemment.

### 2. Cytotoxicité préalable

La cytotoxicité des composés est évaluée sur les mêmes cellules fibroblastiques sur 5 jours par un test au MTT et par analyse morphologique des tapis cellulaires.

| | |
|---|---|
| - plaques : | 48 puits |
| - pré-culture : | 24h |
| - cellules/puits (1 cm²) : | 10^4 cellules/cm² |
| - réplicates : | 6 |
| - temps de contact : | 5 jours |
| - paramètres : | MTT et morphologiques |

### 3. Traitement et dosage du pro-collagène I

Les fibroblastes sont ensemencés (JO), à la même densité cellulaire que pour la cytotoxicité préalable, en milieu DMEM à 10%, et incubés à 37°C et 5% de CO₂. Après adhésion des cellules (J1), le milieu de culture est éliminé puis remplacé par du milieu contenant ou non (témoin) les produits à l'essai. Le milieu DMEM est alors à 1% de SVF pour éviter le signal de base trop élevé dû à la présence de sérum.

L'association Adénosine / Rhamnose est utilisée respectivement à la dose de 10µM et 1 Mm dans le puits.

Les cellules sont incubées à 37°C pendant 5 jours afin d'observer une réponse générale des fibroblastes dans leur production de procollagène I.

Chaque condition expérimentale est réalisée en triplicata.

Après incubation, les milieux de culture ont été prélevés et la quantité de procollagène I présente dans les milieux de culture a été mesurée à l'aide d'un kit de dosage ELISA spécifique (Procollagen Type I C-Peptide EIA Kit, Bio-Whittaker MK101).

Une observation de la morphologie cellulaire et un test de viabilité au MTT ont été réalisés sur les tapis cellulaires correspondants.

### Résultats

L'association Rhamnose / adénosine la production de procollagène I par les fibroblastes dermiques.

### Exemple 5 : exemple de réalisation d'une composition cosmétique selon l'invention

| ***Crèmes ANTI Age Totale : émulsion huile dans eau*** | |
|---|---|
| Ammonium Polyacryldimethyltauramide (Hostacerin AMPS de Clariant) | 1,00% |
| Cyclohexasiloxane | 5,0% |
| Huile d'amande d'abricot | 7% |
| Isononyl isononanoate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20 % |
| rhamnose | 5% |
| Adénosine | 0,1% |
| Conservateurs | 0,3% |
| Eau | qsp 100 |

Appliquée bi-quotidiennement pendant 6 mois, une amélioration globale de l'âge apparent du visage, via en particulier une réduction de l'apparence des rides d'expression et une amélioration de l'éclat du teint.

### Exemple 6 : exemple de réalisation d'une composition cosmétique selon l'invention

| ***Crèmes Anti Age : émulsion huile dans eau*** | |
|---|---|
| Ammonium Polyacryldimethyltauramide (Hostacerin AMPS de Clariant) | 1,00% |
| Cyclohexasiloxane | 5,0% |
| Huile d'amande d'abricot | 7% |
| Isononyl isononanoate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20 % |
| **mannose** | 5% |
| **Adénosine** | 0,1% |
| Conservateurs | 0,50% |
| Eau | qsp 100 |

### Exemple 7 : exemple de réalisation d'une composition cosmétique selon l'invention

| ***Crèmes Anti Age : émulsion huile dans eau*** | |
|---|---|
| Ammonium Polyacryldimethyltauramide (Hostacerin AMPS de Clariant) | 1,00% |
| Cyclohexasiloxane | 5,0% |
| Huile d'amande d'abricot | 7% |
| Isononyl isononanoate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20 % |
| **Mannose** | 2,5% |
| **Rhamnose** | 2,5% |
| **Adénosine** | 0,1% |
| Conservateurs | 0,50% |
| Eau | qsp 100 |

### Exemple 8 : exemple de réalisation d'une composition cosmétique selon l'invention

Crème de jour anti-âge pour le visage

**Phase A1 :**

| | |
|---|---|
| - Distéarate de sucrose commercialisé par la Société STEARINERIE DUBOIS | 1,75% |
| - Stéarate de sorbitane oxyéthyléné à 4 moles d'oxyde d'éthylène commercialisé par la Société ICI | |
| sous le nom "TWEEN 61" | 1,15% |
| - Acide stéarique | 0,75% |
| - Stearyl heptanoate | 4,00% |
| - Vaseline codex | 1,50% |
| - Huiled'avocat | 3,20% |
| - Huile de jojoba | 3,00% |
| - Huile de silicone volatile | 2,70% |
| - Acétate de vitamine E | 1,00% |
| - Glycérides de Vitamine F | 3,00% |

**Phase A2 :**

| | |
|---|---|
| Gomme de silicone commercialisée par DOW CORNING sous le nom "Q2-1403 Fluid" | 3,00% |
| - Propylparaben | 0,2% |
| Parfum | 0,3% |

**Phase B :**

| | | |
|---|---|---|
| Glycérine | | 3,00% |
| Hydroxyproline | | 1,00% |
| D-panthenol | | 1,00% |
| Triéthanolamine | | 0,35% |
| Rhamnose | | 3,00% |
| Adénosine | | 0.2% |
| Méthylparaben | | 0,3% |
| Eau déminéralisée | q.s.p. | 100% |

**Phase C :**

| | |
|---|---|
| Ammonium Polyacryldimethyltauramide (Hostacerin AMPS de Clariant) | 1% |

## Revendications

1. Composition cosmétique comprenant, dans un milieu physiologiquement acceptable, l'association d'au moins un monosaccharide choisi parmi le mannose, le rhamnose et leur mélange, et d'au moins un composé additionnel choisi parmi l'adénosine, un de ses analogues et leur mélange, dans laquelle la quantité dudit composé additionnel est comprise entre 0,01 % et 10 % en poids par rapport au poids total de la composition,
et dans laquelle l'adénosine ou un de ses analogues est choisi parmi l'adénosine, la 2'-deoxyadénosine, la 2', 3'-isopropylidene adénosine, la toyocamycine, la 1-méthyladénosine, la N-6-methyladénosine, l'adénosine N-oxyde, le 6-méthylmercaptopurine riboside, le 6-4 chloropurine riboside, la phénylisopropyl-adénosine ("PIA"), la 1-méthylisoguanosine, la N6-cyclohexyladénosine (CHA), la N6-cyclopentyladénosine (CPA), la 2-chloro-N6-cyclopentyladénosine, la 2-chloroadénosine, la N6-phényladénosine, la 2-phénylaminoadénosine, la MECA, la N6-phénéthyladénosine, la 2-p-(2-carboxy-éthyl) phénéthyl-amino-5'-N-éthylcarboxamido-adénosine (CGS-21680), la N éthylcarboxamido-adénosine (NECA), la 5' (N-cyclopropyl)-carboxamidoadénosine, la DPMA (PD 129,944), le metrifudil, l'érythro-9-(2-hydroxy-3-nonyl) adénine ("EHNA"), l'iodotubercidine, les composés de formule (I): 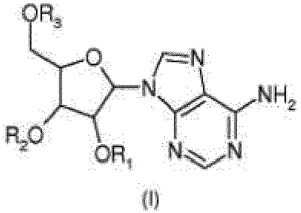 dans laquelle :
R1 et R2 identiques désignent un radical hydrocarboné linéaire saturé en C1-C6 ou insaturé en C2-C6, ou ramifié en C3-C6 saturé ou insaturé, ou bien encore forment ensemble, avec les atomes d'oxygène auxquels ils sont rattachés, un radical isopropylidène ;
R3 désigne :
(i) un radical hydrocarboné linéaire saturé en C1-C10 ou insaturé en C2-C10, ou ramifié en C3-C10 saturé ou insaturé, éventuellement substitué par au moins un groupement choisi parmi -OR', -NR'R", -COOR', -CONR'R", -CF3, -F, -OCF3, -CN, -NO2, ou
(ii) un groupe -COR4 avec R4 désignant un radical hydrocarboné linéaire saturé en C1-C9 ou insaturé en C2-C9, ou ramifié en C3-C9 saturé ou insaturé, éventuellement substitué par au moins un groupement choisi parmi -OR', -NR'R", -COOR', -CONR'R", -CF3, -F, -OCF3, -CN, -NO2 ou
(iii) un groupe ester issu de la biotine;
R' et R" désignant un atome d'hydrogène, un radical hydrocarboné linéaire saturé en C1-C6 ou insaturé en C2-C6, ou ramifié en C3-06 saturé ou insaturé, éventuellement substitué par au moins un groupement choisi parmi
-OZ, -NZZ', -COOZ, Z et Z' désignant, indépendamment l'un de l'autre, un atome d'hydrogène ou un radical hydrocarboné linéaire saturé en C1-C6 ou insaturé en C2-C6, ou ramifié en C3-C6 saturé ou insaturé ; et
leurs sels, isomères optiques et solvates.

2. Composition selon la revendication 1, comprenant en outre au moins un sel de magnésium et au moins un sel de potassium.

3. Composition selon la revendication 2, **caractérisée en ce que** ledit sel de magnésium est le sulfate de magnésium.

4. Composition selon la revendication 2, **caractérisée en ce que** ledit sel de potassium est le glycyrrhizinate dipotassique.

5. Composition selon l'une quelconque des revendications précédentes, **caractérisée en ce que** lesdits analogues d'adénosine sont choisis dans le groupe constitué de la 2'-deoxyadénosine, la 2',3'-isopropoylidene adénosine, la toyocamycine, la 1-méthyladénosine, la N-6-méthyladénosine, l'adénosine N-oxyde, le 6-méthylmercaptopurine riboside et le 6-4 chloropurine riboside.

6. Composition selon l'une quelconque des revendications 1 à 4, **caractérisée en ce que** lesdits analogues d'adénosine sont choisis dans le groupe constitué de la phénylisopropyl-adénosine ("PIA"), la 1-méthylisoguanosine, la N6-Cyclohexyladénosine (CHA), la N6-cyclopentyladénosine (CPA), la 2-chloro-N6-cyclopentyladénosine, la 2-chloroadénosine, la N6-phényladénosine, la 2-phénylaminoadénosine, la MECA, la N6-phénéthyladénosine, la 2-p-(2-carboxyéthyl)phénéthyl-amino-5'-N-éthylcarboxamido-adénosine (CGS-21680), la N éthylcarboxamido-adénosine (NECA), la 5' (N-cyclopropyl)-carboxamidoadénosine, la DPMA (PD 129,944) et le metrifudil.

7. Composition selon l'une quelconque des revendications 1 à 6, **caractérisée en ce que** lesdits dérivés d'adénosine sont choisis dans le groupe constitué de l'érythro-9-(2-hydroxy-3-nonyl) adénine ("EHNA") et l'iodotubercidine.

8. Composition selon l'une quelconque des revendications 1 à 6, **caractérisée en ce que** lesdits analogues d'adénosine sont choisis parmi des composés de formule (I) suivante : 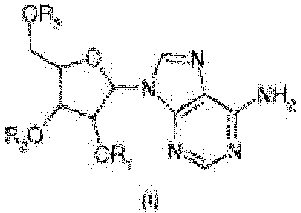 dans laquelle :
R1 et R2 identiques désignent un radical hydrocarboné linéaire saturé en C1-C6 ou insaturé en C2-C6, ou ramifié en C3-C6 saturé ou insaturé, ou bien encore forment ensemble, avec les atomes d'oxygène auxquels ils sont rattachés un radical isopropylidène ;
R3 désigne :
(i) un radical hydrocarboné linéaire saturé en C1-C10 ou insaturé en C2-C10, ou ramifié en C3-C10 saturé ou insaturé, éventuellement substitué par au moins un groupement choisi parmi -OR', -NR'R", -COOR', -CONR'R", -CF3,
-F, -OCF3, -CN, -NO2, ou
(ii) un groupe -COR4 avec R4 désignant un radical hydrocarboné linéaire saturé en C1-C9 ou insaturé en C2-C9, ou ramifié en C3-C9 saturé ou insaturé, éventuellement substitué par au moins un groupement choisi parmi -OR', -NR'R", -COOR', -CONR'R", -CF3, -F , -OCF3, -CN, -NO2 ou
(iii) un groupe ester issu de la biotine;
R' et R" désignant un atome d'hydrogène, un radical hydrocarboné linéaire saturé en C1-C6 ou insaturé en C2-C6, ou ramifié en C3-06 saturé ou insaturé, éventuellement substitué par au moins un groupement choisi parmi
-OZ, -NZZ', -COOZ, Z et Z' désignant, indépendamment l'un de l'autre, un atome d'hydrogène ou un radical hydrocarboné linéaire saturé en C1-C6 ou insaturé en C2-C6, ou ramifié en C3-C6 saturé ou insaturé ; et
leurs sels, isomères optiques et solvates.

9. Composition selon l'une quelconque des revendications 1 à 4, **caractérisée en ce que** ledit composé additionnel est l'adénosine.

10. Composition selon l'une quelconque des revendications précédentes, dans laquelle la quantité desdits monosaccharides est comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

11. Composition selon l'une quelconque des revendications précédentes, **caractérisée en ce qu'**elle est adaptée à une administration topique sur la peau ou ses phanères, une administration orale ou une injection cutanée.

12. Utilisation cosmétique de l'association d'au moins un monosaccharide choisi parmi le mannose, le rhamnose et leur mélange avec un composé additionnel tel que défini dans la revendication 1, pour diminuer et/ou prévenir les signes du vieillissement de la peau ou de ses phanères.

13. Utilisation selon la revendication 12, pour diminuer et/ou prévenir les caractéristiques des rides et/ou ridules.

14. Dispositif comprenant au moins, l'association d'au moins un monosaccharide choisi parmi le mannose, le rhamnose et leur mélange, et d'au moins un composé additionnel choisi parmi l'adénosine, un de ses analogues et leur mélange, le dispositif étant adapté à une injection intraépidermique et/ou intradermique et/ou sous-cutanée.

## Patentansprüche

1. Kosmetische Zusammensetzung, umfassend in einem physiologisch verträglichen Medium die Kombination aus wenigstens einem Monosaccharid, ausgewählt aus Mannose, Rhamnose und deren Gemisch, und wenigstens einer zusätzlichen Verbindung, ausgewählt aus Adenosin, einem seiner Analoga und deren Gemisch, wobei die Menge besagter zusätzlicher Verbindung zwischen 0,01 Gew.-% und 10 Gew.-% bezogen auf das Gesamtgewicht der Zusammensetzung beträgt,
und wobei Adenosin oder eines seiner Analoga ausgewählt ist aus Adenosin, 2'-Desoxyadenosin, 2`,3`-Isopropylidenadenosin, Toyocamycin, 1-Methyladenosin, N6-Methyladenosin, Adenosin-N-Oxid, 6-Methylmercaptopurinribosid, 6-4-Chéloipurinubosid, Phenylisopropyladenosin ("PIA"), 1-Methylisoguanosin, N6-Cyclohexyladenosin (CHA), N6-Cyclopentyladenosin (CPA), 2-Chlor-N6-cyclopentyladenosin, 2-Chloradenosin, N6-Phenyladenosin, 2-Phenylaminoadenosin, MECA, N6-Phenethyladenosin, 2-p-(2-Carboxy-ethyl)-phenethyl-amino-5`-N-ethylcarboxamido-adenosin (CGS-21680), N-Ethylcarboxamidoadenosin (NECA), 5'-(N-Cyclopropyl)-carboxamidoadenosin, DPMA (PD 129.944), Metrifudil, Erythro-9-(2-hydroxy-3-nonyl)-adenin ("EHNA"), Iodtubercidin, den Verbindungen der Formel (I): 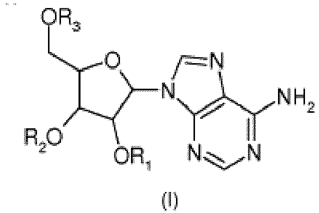 wobei:
R1 und R2 identisch sind und einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen oder zusammen mit den Sauerstoffatomen, an die sie gebunden sind, einen Isopropylidenrest bilden;
R3 bezeichnet:
(i) einen linearen gesättigten C1-C10-Kohlenwasserstoffrest oder ungesättigten C2-C10-Kohlenwasserstoffrest oder verzweigten gesättigten oder ungesättigten C3-C10-Kohlenwasserstoffrest, gegebenenfalls substituiert mit wenigstens einer Gruppe, ausgewählt aus: -OR', -NR'R", COOR', -CONR'R", -CF3, -F, -OCF3, -CN, -NO2, oder
(ii) eine Gruppe -COR4, wobei R4 einen linearen gesättigten C1-C9-Kohlenwasserstoffrest oder ungesättigten C2-C9-Kohlenwasserstoffrest oder verzweigten gesättigten oder ungesättigten C3-C9-Kohlenwasserstoffrest bezeichnet, gegebenenfalls substituiert mit wenigstens einer Gruppe, ausgewählt aus: -OR', -NR'R", COOR', -CONR'R", -CF3, -F, -OCF3, -CN, -NO2; oder
(iii) eine von Biotin stammende Ester-Gruppe;
wobei R' und R" ein Wasserstoffatom, einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen, gegebenenfalls substituiert mit wenigstens einer Gruppe, ausgewählt aus:
-OZ, -NZZ`, -COOZ, wobei Z und Z' unabhängig voneinander ein Wasserstoffatom, einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen; und
Salze, optische Isomere und Solvate davon.

2. Zusammensetzung gemäß Anspruch 1, umfassend außerdem wenigstens ein Magnesiumsalz und wenigstens ein Kaliumsalz.

3. Zusammensetzung gemäß Anspruch 2, **dadurch gekennzeichnet, dass** besagtes Magnesiumsalz Magnesiumsulfat ist.

4. Zusammensetzung gemäß Anspruch 2, **dadurch gekennzeichnet, dass** besagtes Kaliumsalz Dikaliumglycyrrhizinat ist.

5. Zusammensetzung gemäß einem der vorhergehenden Ansprüche, **dadurch gekennzeichnet, dass** besagte Adenosin-Analoga aus der Gruppe ausgewählt sind, bestehend aus 2'-Desoxyadenosin, 2',3'-Isopropylidenadenosin, Toyocamycin, 1-Methyladenosin, N6-Methyladenosin, Adenosin-N-Oxid, 6-Methylmercaptopurinribosid und 6-4-Chlorpurinribosid.

6. Zusammensetzung gemäß einem der Ansprüche 1 bis 4, **dadurch gekennzeichnet, dass** besagte Adenosin-Analoga aus der Gruppe ausgewählt sind, bestehend aus Phenylisopropyladenosin ("PIA"), 1-Methylisoguanosin, N6-Cyclohexyladenosin (CHA), N6-Cyclopentyladenosin (CPA), 2-Chlor-N6-cyclopentyladenosin, 2-Chloradenosin, N6-Phenyladenosin, 2-Phenylaminoadenosin, MECA, N6-Phenethyladenosin, 2-p-(2-Carboxy-ethyl)-phenethyl-amino-5 `-N-ethylcarboxamido-adenosin (CGS-21680), N-Ethylcarboxamido-adenosin (NECA), 5'-(N-Cyclopropyl)-carboxamidoadenosin, DPMA (PD 129.944) und Metrifudil.

7. Zusammensetzung gemäß einem der Ansprüche 1 bis 6, **dadurch gekennzeichnet, dass** besagte Adenosin-Analoga aus der Gruppe ausgewählt sind, bestehend aus Erythro-9-(2-hydroxy-3-nonyl)-adenin ("EHNA") und Iodtubercidin.

8. Zusammensetzung gemäß einem der Ansprüche 1 bis 6, **dadurch gekennzeichnet, dass** besagte Adenosin-Analoga aus Verbindungen der nachfolgenden Formel (I) ausgewählt sind: 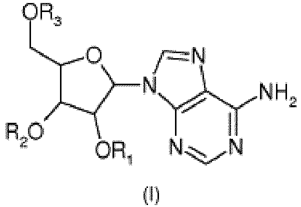 wobei:
R1 und R2 identisch sind und einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen oder zusammen mit den Sauerstoffatomen, an die sie gebunden sind, einen Isopropylidenrest bilden;
R3 bezeichnet:
(i) einen linearen gesättigten C1-C10-Kohlenwasserstoffrest oder ungesättigten C2-C10-Kohlenwasserstoffrest oder verzweigten gesättigten oder ungesättigten C3-C10-Kohlenwasserstoffrest, gegebenenfalls substituiert mit wenigstens einer Gruppe, ausgewählt aus: -OR', -NR'R", COOR', -CONR'R", -CF3, -F, -OCF3, -CN, -NO2, oder
(ii) eine Gruppe -COR4, wobei R4 einen linearen gesättigten C1-C9-Kohlenwasserstoffrest oder ungesättigten C2-C9-Kohlenwasserstoffrest oder verzweigten gesättigten oder ungesättigten C3-C9-Kohlenwasserstoffrest bezeichnet, gegebenenfalls substituiert mit wenigstens einer Gruppe, ausgewählt aus: -OR', -NR'R", COOR', -CONR'R", -CF3, -F, -OCF3, -CN, -NO2; oder
(iii) eine von Biotin stammende Ester-Gruppe;
wobei R' und R" ein Wasserstoffatom, einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen, gegebenenfalls substituiert mit wenigstens einer Gruppe, ausgewählt aus:
-OZ, -NZZ`, -COOZ, wobei Z und Z' unabhängig voneinander ein Wasserstoffatom, einen linearen gesättigten C1-C6-Kohlenwasserstoffrest oder ungesättigten C2-C6-Kohlenwasserstoffrest oder verzweigten gesättigten oder ungesättigten C3-C6-Kohlenwasserstoffrest bezeichnen; und
Salze, optische Isomere und Solvate davon.

9. Zusammensetzung gemäß einem der Ansprüche 1 bis 4, **dadurch gekennzeichnet, dass** besagte zusätzliche Verbindung Adenosin ist.

10. Zusammensetzung gemäß einem der vorhergehenden Ansprüche, wobei die Menge an besagten Monosacchariden zwischen 0,001 Gew.-% und 30 Gew.-% bezogen auf das Gesamtgewicht der Zusammensetzung und insbesondere zwischen 0,1 Gew.-% und 10 Gew.-% und ganz besonders zwischen 0,5 Gew.-% und 6 Gew.-% bezogen auf das Gesamtgewicht der Zusammensetzung beträgt.

11. Zusammensetzung gemäß einem der vorhergehenden Ansprüche, **dadurch gekennzeichnet, dass** sie für eine topische Verabreichung auf die Haut oder ihre Hautanhangsgebilde, für eine orale Verabreichung oder für eine kutane Injektion geeignet ist.

12. Kosmetische Verwendung der Kombination aus wenigstem einem Monosaccharid, ausgewählt aus Mannose, Rhamnose und deren Gemisch, mit einer wie in Anspruch 1 definierten zusätzlichen Verbindung zur Verringerung und/oder Prävention von Anzeichen der Alterung der Haut oder ihren Hautanhangsgebilden.

13. Verwendung gemäß Anspruch 12 zur Verringerung und/oder Prävention der Charakteristika von Falten und/oder Fältchen.

14. Vorrichtung, umfassend wenigstens die Kombination aus wenigstens einem Monosaccharid, ausgewählt aus Mannose, Rhamnose und deren Gemisch, und wenigstens einer zusätzlichen Verbindung, ausgewählt aus Adenosin, einem seiner Analoga und deren Gemisch, wobei die Vorrichtung für eine intraepidermale und/oder intradermale und/oder subkutane Injektion geeignet ist.

## Claims

1. Cosmetic composition comprising, in a physiologically acceptable medium, a combination of at least one monosaccharide chosen from mannose, rhamnose and their mixture, and of at least one additional compound chosen from adenosine, an analogue thereof and a mixture thereof, in which the amount of the said additional compound is between 0.01% and 10% by weight relative to the total weight of the compositionat,
and in which the adenosine or analogue thereof is chosen from adenosine, 2'-deoxyadenosine, 2',3'-isopropylideneadenosine, toyocamycine, 1-methyladenosine, N-6-methyladenosine, adenosine N-oxide, 6-methylmercaptopurine riboside, 6-4 chloropurine riboside, phenylisopropyladenosine (PIA), 1-methylisoguanosine, N6-cyclohexyladenosine (CHA), N6-cyclopentyladenosine (CPA), 2-chloro-N6-cyclopentyladenosine, 2-chloroadenosine, N6-phenyladenosine, 2-phenylaminoadenosine, MECA, N6-phenethyladenosine, 2-p-(2-carboxyethyl)phenethylamino-5'-N-ethylcarboxamidoadenosine (CGS-21680), N-ethylcarboxamidoadenosine (NECA), 5'-(N-cyclopropyl)carboxamidoadenosine, DPMA (PD129,944), metrifudil, erythro-9-(2-hydroxy-3-nonyl)adenine (EHNA), iodotubercidine, the compounds of formula (I): 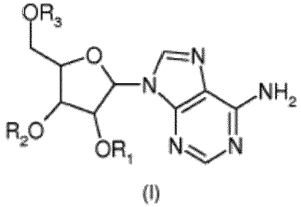 in which:
R1 and R2, which are identical, denote a linear saturated C1-C6 or unsaturated C2-C6, or branched saturated or unsaturated C3-C6 hydrocarbon-based radical, or alternatively form, together with the oxygen atoms to which they are attached, an isopropylidene radical;
R3 denotes:
(i) a linear saturated C1-C10 or unsaturated C2-C10, or branched saturated or unsaturated C3-C10 hydrocarbon-based radical, optionally substituted with at least one group chosen from -OR', -NR'R", -COOR', CONR'R", -CF₃, -F, -OCF₃, -CN and - NO₂, or
(ii) a group -COR4 with R4 denoting a linear saturated C1-C9 or unsaturated C2-C9, or branched saturated or unsaturated C3-C9 hydrocarbon-based radical, optionally substituted with at least one group chosen from -OR', -NR'R", -COOR', - CONR'R", -CF₃, -F, -OCF₃, -CN and -NO₂; or
(iii) a biotin-based ester group;
R' and R" denoting a hydrogen atom, a linear saturated C1-C6 or unsaturated C2-C6, or branched saturated or unsaturated C3-C6 hydrocarbon-based radical, optionally substituted with at least one group chosen from -OZ, -NZZ' and -COOZ, Z and Z' denoting, independently of each other, a hydrogen atom or a linear saturated C1-C6 or unsaturated C2-C6, or branched saturated or unsaturated C3-C6 hydrocarbon-based radical; and
the salts, optical isomers and solvates thereof.

2. Composition according to Claim 1, also comprising at least one magnesium salt and at least one potassium salt.

3. Composition according to Claim 2, **characterized in that** the said magnesium salt is magnesium sulfate.

4. Composition according to Claim 2, **characterized in that** the said potassium salt is dipotassium glycyrrhizinate.

5. Composition according to any one of the preceding claims, **characterized in that** the said adenosine analogues are chosen from the group formed by 2'-deoxyadenosine, 2',3'-isopropylideneadenosine, toyocamycin, 1-methyladenosine, N-6-methyladenosine, adenosine N-oxide, 6-methylmercaptopurine riboside and 6-4 chloropurine riboside.

6. Composition according to any one of Claims 1 to 4, **characterized in that** the said adenosine analogues are chosen from the group formed by phenylisopropyladenosine (PIA), 1-methylisoguanosine, N6-cyclohexyladenosine (CHA), N6-cyclopentyladenosine (CPA), 2-chloro-N6- cyclopentyladenosine, 2-chloroadenosine, N6-phenyladenosine, 2-phenylaminoadenosine, MECA, N6-phenethyladenosine, 2-p-(2-carboxyethyl)phenethylamino-5'-N-ethylcarboxamidoadenosine (CGSZ-21680), N-ethylcarboxamidoadenosine (NECA), 5'-(N-cyclopropyl)carboxamidoadenosine, DPMA (PD 129,944) and metrifudil.

7. Composition according to any one of Claims 1 to 6, **characterized in that** the said adenosine derivatives are chosen from the group formed by erythro-9-(2-hydroxy-3-nonyl)adenine (EHNA) and iodotubercidine.

8. Composition according to any one of Claims 1 to 6, **characterized in that** the said adenosine analogues are chosen from compounds of formula (I) below: 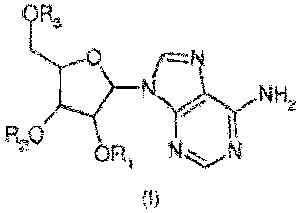 in which:
R1 and R2, which are identical, denote a linear saturated C1-C6 or unsaturated C2-C6, or branched saturated or unsaturated C3-C6 hydrocarbon-based radical, or alternatively form, together with the oxygen atoms to which they are attached, an isopropylidene radical;
R3 denotes:
(i) a linear saturated C1-C10 or unsaturated C2-C10, or branched saturated or unsaturated C3-C10 hydrocarbon-based radical, optionally substituted with at least one group chosen from -OR', -NR'R", -COOR', CONR'R", -CF₃, -F, -OCF₃, -CN and - NO₂, or
(ii) a group -COR4 with R4 denoting a linear saturated C1-C9 or unsaturated C2-C9, or branched saturated or unsaturated C3-C9 hydrocarbon-based radical, optionally substituted with at least one group chosen from -OR', -NR'R", -COOR', - CONR'R", -CF₃, -F, -OCF₃, -CN and -NO₂; or
(iii) a biotin-based ester group;
R' and R" denoting a hydrogen atom, a linear saturated C1-C6 or unsaturated C2-C6, or branched saturated or unsaturated C3-C6 hydrocarbon-based radical, optionally substituted with at least one group chosen from -OZ, -NZZ' and -COOZ, Z and Z' denoting, independently of each other, a hydrogen atom or a linear saturated C1-C6 or unsaturated C2-C6, or branched saturated or unsaturated C3-C6 hydrocarbon-based radical; and
the salts, optical isomers and solvates thereof.

9. Composition according to any one of Claims 1 to 4, **characterized in that** the said additional compound is adenosine.

10. Composition according to anyone of the preceding claims, in which the amount of the said monosaccharides is between 0.001% and 30% by weight relative to the total weight of the composition, preferably between 0.1 % and 10% by weight, and more preferably between 0.5% and 6% by weight relative to the total weight of the composition.

11. Composition according to anyone of the preceding claims, **characterized in that** it is suitable for topical administration to the skin or its integuments, oral administration or cutaneous injection.

12. Cosmetic use of a combination of at least one monosaccharide chosen from mannose, rhamnose and their mixture, with an additional compound as defined in claim 1, for reducing and/or preventing the signs of ageing of the skin or its integuments.

13. Use according to Claim 12, for reducing and/or preventing the characteristics of wrinkles and/or fine lines.

14. Device comprising at least a combination of at least one monosaccharide chosen from mannose, rhamnose and their mixture, and of at least one additional compound chosen from adenosine, an analogue thereof and a mixture thereof,
the device being suitable for intraepidermal and/or intradermal and/or subcutaneous injection.
